# EUROPÄISCHE PATENTSCHRIFT

(11) **EP 3 351 110 B1**
(45) Veröffentlichungstag und Bekanntmachung des Hinweises auf die Patenterteilung: **04.03.2020**
(21) Anmeldenummer: 17152543.9
(22) Anmeldetag: 22.01.2017
(51) Int. Cl.: A23C 9/142, C07K 1/34, C07K 14/47, C07C 51/47, A23C 21/00, A23C 21/02, A23J 1/20, B01D 61/02, A23C 1/04, A23J 1/08

(54) **VERFAHREN ZUR GEKOPPELTEN GEWINNUNG VON SÜSSMOLKE UND MILCHSÄURE AUS SAUERMOLKE**
PROCESS FOR COUPLED PRODUCTION OF SWEET WHEY AND LACTIC ACID FROM ACID WHEY
PROCÉDÉ POUR LA PRODUCTION ACCOMPAGNÉE DU LACTOSÉRUM DOUX ET D'ACIDE LACTIQUE À PARTIR DE LACTOSÉRUM ACIDE

(43) Veröffentlichungstag der Anmeldung: 25.07.2018
(73) Patentinhaber: DMK Deutsches Milchkontor GmbH, 27404 Zeven (DE)
(72) Erfinder: DÖRING, Sven-Rainer, 27404 Zeven (DE); HUNOLD, Mareike, 27404 Zeven (DE)
(74) Vertreter: Fabry, Bernd

(56) Entgegenhaltungen:
- WO-A1-2014/163486
- GB-A- 2 293 825
- US-A1- 2014 348 981
- BARRANTES L D ET AL: "PARTIAL DEACIDIFICATION AND DEMINERALIZATION OF COTTAGE CHEESE WHEYBY NANOFILTRATION", JOURNAL OF FOOD SCIENCE, WILEY-BLACKWELL PUBLISHING, INC, US, Bd. 62, Nr. 2, 1. März 1997 (1997-03-01), Seiten 338-341, XP000691229, ISSN: 0022-1147, DOI: 10.1111/J.1365-2621.1997.TB03996.X
- YEBO LI ET AL: "Separate and Concentrate Lactic Acid Using Combination of Nanofiltration and Reverse Osmosis Membranes", APPLIED BIOCHEMISTRY AND BIOTECHNOLOGY, Bd. 147, Nr. 1-3, 25. September 2007 (2007-09-25), Seiten 1-9, XP055217115, ISSN: 0273-2289, DOI: 10.1007/s12010-007-8047-5
- ROMAN A ET AL: "Partial demineralization and concentration of acid whey by nanofiltration combined with diafiltration", DESALINATION, ELSEVIER, AMSTERDAM, NL, Bd. 241, Nr. 1-3, 31. Mai 2009 (2009-05-31), Seiten 288-295, XP026012024, ISSN: 0011-9164, DOI: 10.1016/J.DESAL.2007.12.054 [gefunden am 2009-03-19]
- TIMMER J M K ET AL: "TRANSPORT OF LACTIC ACID THROUGH REVERSE OSMOSIS AND NANOFILTRATIONMEMBRANES", JOURNAL OF MEMBRANE SCIENCE, ELSEVIER BV, NL, Bd. 85, Nr. 2, 1. November 1993 (1993-11-01), Seiten 205-216, XP000490124, ISSN: 0376-7388, DOI: 10.1016/0376-7388(93)85169-W
- GONZALEZ M I ET AL: "Lactic acid recovery from whey ultrafiltrate fermentation broths and artificial solutions by nanofiltration", DESALINATION, ELSEVIER, AMSTERDAM, NL, Bd. 228, Nr. 1-3, 15. August 2008 (2008-08-15), Seiten 84-96, XP022735174, ISSN: 0011-9164, DOI: 10.1016/J.DESAL.2007.08.009 [gefunden am 2008-06-18]
- CHANDRAPALA JAYANI ET AL: "Strategies for maximizing removal of lactic acid from acid whey - Addressing the un-processability issue", SEPARATION AND PURIFICATION TECHNOLOGY, ELSEVIER SCIENCE, AMSTERDAM, NL, Bd. 172, 6. September 2016 (2016-09-06), Seiten 489-497, XP029755652, ISSN: 1383-5866, DOI: 10.1016/J.SEPPUR.2016.09.004
- CHANDRAPALA JAYANI ET AL: "Nanofiltration and nanodiafiltration of acid whey as a function of pH and temperature", SEPARATION AND PURIFICATION TECHNOLOGY, ELSEVIER SCIENCE, AMSTERDAM, NL, Bd. 160, 29. Dezember 2015 (2015-12-29), Seiten 18-27, XP029405692, ISSN: 1383-5866, DOI: 10.1016/J.SEPPUR.2015.12.046
- PAN K ET AL: "A study of demineralization of whey by nanofiltration membrane", DESALINATION, ELSEVIER, AMSTERDAM, NL, Bd. 267, Nr. 2-3, 15. Februar 2011 (2011-02-15), Seiten 217-221, XP027567843, ISSN: 0011-9164 [gefunden am 2010-10-30]
- BOGDAN DEC ET AL: "CHARACTERISTICS OF ACID WHEY POWDER PARTIALLY DEMINERALISED BY NANOFILTRATION", POLISH JOURNAL OF FOOD AND NUTRITION SCIENCES, Bd. 15, Nr. 56, 1. Januar 2006 (2006-01-01), Seiten 87-90, XP055364283,
- JINDAL A R ET AL: "PREPARATION AND COMPOSITION OF CHHANA WHEY POWDERS USING MEMBRANE PROCESSING TECHNIQUES", JOURNAL OF THE SCIENCE OF FOOD AND AGRICULTURE, WILEY & SONS, CHICHESTER, GB, Bd. 58, Nr. 4, 1. Januar 1992 (1992-01-01) , Seiten 511-517, XP000275250, ISSN: 0022-5142
- TIMMER J M K ET AL: "Lactic acid separation from fermentation broths by reverse osmosis and nanofiltration", JOURNAL OF MEMBRANE SCIENCE, ELSEVIER BV, NL, Bd. 92, 1. Januar 1994 (1994-01-01), Seiten 185-197, XP008128611, ISSN: 0376-7388
- MINH NGUYEN ET AL: "By-product recovery from cottage cheese production by nanofiltration", JOURNAL OF CLEANER PRODUCTION, Bd. 11, Nr. 7, 1. November 2003 (2003-11-01), Seiten 803-807, XP055364619, AMSTERDAM, NL ISSN: 0959-6526, DOI: 10.1016/S0959-6526(02)00130-0

## Beschreibung

### GEBIET DER ERFINDUNG

Die Erfindung befindet sich auf dem Gebiet der Milchverarbeitung und betrifft ein Verfahren, mit dessen Hilfe man Sauermolke in Süßmolke umwandeln kann und dabei als wertvolles Koppelprodukt Milchsäure gewinnt.

### STAND DER TECHNIK

Molke ist die wässrige grünlich-gelbe Restflüssigkeit, die bei der Käseherstellung entsteht. Sie ist der flüssige Teil, der nach der Gerinnung der Milch zu Käse oder Quark abgesondert wird. Je nach Herstellungsverfahren unterscheidet man zwei Typen: die *Süßmolke* (auch *Labmolke*), die entsteht, wenn man Milch mit Lab zur Käseherstellung dicklegt, und die *Sauermolke,* die entsteht, wenn Milch durch Milchsäurebakterien fermentiert wird. Molke besteht zu 94 Gew.-% aus Wasser, zu 4 bis 5 Gew.-%% aus Lactose und ist nahezu fettfrei. Außerdem enthält sie Milchsäure, die Vitamine B₁, B₂ (dies bewirkt die grünliche Farbe) und B₆ sowie Kalium, Calcium, Phosphor und andere Mineralstoffe, doch vor allem 0,6 bis 1 Gew.-% Molkenprotein. Molke enthält damit deutlich weniger Eiweiß als Milch. Insbesondere enthält sie anders als Milch kein Kasein. Süßmolke weist einen fast neutralen pH-Wert auf, Sauermolke besitzt dahingegen einen pH-Wert von 5 oder weniger.

Süßmolke ist als die wichtigste Molkenform anzusehen und dient sowohl als Rohstoffquelle für weitere Milchprodukte, vor allem für die so genannten Molkenproteine, wird jedoch auch im Nahrungsmittelbereich eingesetzt.

Sauermolke stellt hingegen ein Abfallprodukt dar, das in großen Mengen bei der Frischkäserei und Quarkherstellung anfällt. Wegen des hohen Milchsäureanteils und des damit verbundenen niedrigen pH-Wertes ist es für die menschliche Ernährung nur mit Einschränkungen zu verwenden. Sauermolke erweist sich auch als schwer filtrierbar und lässt sich nur unter großen Schwierigkeiten sprühtrocknen. Damit bleibt für dieses Koppelprodukt der Käseherstellung in der Regel nur der Weg der Entsorgung, was zu zusätzlicher Umweltbelastung und Kosten führt und einen Verlust darstellt, da damit auch an sich interessante Inhaltsstoffe der Milch, insbesondere Molkenproteine, Lactose und Calciumphosphat, verloren gehen.

BARRANTES L. D. et al. (J food science, Bd. 62, Nr. 2, 1997) offenbart die Deazidifizierung und Demineralisierung von Hüttenkäsemolke mittels Nanofiltration und Nanodiafiltration.

YEBO L. et al. (Appl. Biochem. and Biotech., Bd. 147, Nr. 1-3, 2007) offenbart die die Trennung und Konzentrierung von Milchsäure aus einer fermentierten ultra-filtrierten Käsemolkepräparats mittels Nanofiltration und Revese-Osmose.

ROMAN A. et al. (Desalination, Bd. 241, Nr. 1-3, 2009) offenbart die partielle Demineralisierung und Konzentrierung von Sauermolke mittels Nanofiltration und Diafiltration.

Die Aufgabe der Erfindung hat somit darin bestanden, Sauermolke in solcher Weise von unerwünschten Bestandteilen zu befreien, dass ein kommerziell attraktives und verwertbares Produkt erhalten wird. Insbesondere bestand Bedarf an einem Produkt, das im Hinblick auf Zusammensetzung und pH-Wert mit einer Süßmolke annähernd identisch ist und Milchsäure als interessanten Industrierohstoff in möglichst konzentrierter und reiner Form als Koppelprodukt liefert.

### BESCHREIBUNG DER ERFINDUNG

Der Gegenstand der Erfindung betrifft ein Verfahren zur gekoppelten Herstellung von Süßmolke und Milchsäure aus Sauermolke, umfassend die folgenden Schritte:
(a) Bereitstellung einer Sauermolke mit einem Milchsäuregehalt von etwa 0,1 bis etwa 1 Gew.-%;
(b) Nanofiltration der Sauermolke unter Gewinnung eines ersten Permeats P1 und eines ersten Retentats R1;
(c) gegebenenfalls Rückverdünnen des ersten Retentats R1 mit Wasser zum Wiedereinstellen der Ausgangstrockenmasse und Vorbereitung des zweiten Nanofiltrationsschrittes;
(d) Nanofiltration des Retentats R1 unter Gewinnung eines zweiten Permeats P2 und einer Süßmolke als zweitem Retentats R2, wobei eine geschlossene NF-Membran mit einer Trenngrenze im Bereich von 150-300 Da eingesetzt wird;
(e) Vereinigen der beiden Permeate P1 und P2 und Unterwerfen der Mischung einer Umkehrosmose, unter Erhalt eines im Wesentlichen nur Wasser enthaltende dritten Permeats P3 und eines Milchsäurekonzentrats als drittes Retentat R3.

Überraschenderweise wurde gefunden, dass sich Sauermolke durch eine zweistufige Nanofiltration in ein Produkt mit Süßmolkenqualität umwandeln lässt, wobei sich Milchsäure in industrieller Reinheit als Koppelprodukt erhalten lässt. Durch Auswahl geeigneter Membranen und Filtrationsbedingungen lässt sich die gewünschte Verarmung der Molke an Milchsäure - und damit natürlich auch die Menge des gewünschten Koppelproduktes - gezielt steuern. Im Rahmen des erfindungsgemäßen Verfahrens findet des Weiteren auch gleichzeitig eine Teildemineralisierung der Ausgangsprodukte statt. Der Demineralisierungsgrad liegt bei 50 bis 70 %. Die Salzfracht wandert in die Milchsäurelösung.

### SAUERMOLKE

Wie eingangs erläutert, stellt Sauermolke die flüssige Phase dar, die bei der Gerinnung von Milch zu Frischkäse oder Quark unter Einsatz von Milchsäurebakterien anfällt. Vorzugsweise werden Qualitäten eingesetzt, die einen Milchsäuregehalt im Bereich von etwa 0,1 bis 1 Gew.-% aufweisen.

### NANOFILTRATION

Die Nanofiltration ist ein Filtrationsverfahren aus dem Bereich der Membrantechnik, mit der sich makromolekulare Substanzen und kleine Partikel aus einem Medium abtrennen und aufkonzentrieren lassen. Man unterscheidet Mikrofiltration, Ultrafiltration und Nanofiltration über den Grad der Abtrennung. Liegt die Ausschlussgrenze (oder auch *"Cut-off"*) bei 100 nm oder darüber, spricht man von Mikrofiltration. Liegt die Ausschlussgrenze in dem Bereich zwischen 2-100 nm, bezeichnet man dies als Ultrafiltration. Bei der Nanofiltration liegt die Ausschlussgrenze unterhalb von 2 nm. In jedem dieser Fälle handelt es sich um rein physikalische, d.h. mechanische Membrantrennverfahren, die nach Prinzip des mechanischen Größenausschlusses arbeiten: alle Partikel in den Fluiden, die größer als die Membranporen sind, werden von der Membran zurückgehalten. Treibende Kraft in beiden Trennverfahren ist der Differenzdruck zwischen Zulauf und Ablauf der Filterfläche, der zwischen 1 und 40 bar liegt, teilweise bis zu 120 bar.

Die Ausschlussgrenzen von Nanofiltrationsmembranen werden auch in Form des *NMWC* (englisch: Nominal Molecular Weight Cut-Off, auch *MWCO,* Molecular Weight Cut Off, Einheit: Dalton) angegeben. Er ist definiert als die minimale Molekülmasse globulärer Moleküle, welche durch die Membran zu 90% zurückgehalten werden. In der Praxis sollte der NMWC mindestens 20 % niedriger sein als die Molmasse des abzutrennenden Moleküls. Weitere qualitative Aussagen über die Filtration lassen sich anhand des *Flux* (Wasserwert) (Transmembranfluss oder Durchtrittsrate) machen. Dieser verhält sich im Idealfall proportional zum Transmembrandruck und reziprok zum Membranwiderstand. Diese Größen werden sowohl von den Eigenschaften der verwendeten Membran als auch durch Konzentrationspolarisation und eventuell auftretendes Fouling bestimmt. Die Durchtrittsrate wird auf 1 m² Membranfläche bezogen. Ihre Einheit ist l/(m²h bar).

Im Hinblick auf eine möglichst effiziente Abtrennung der Milchsäure in zwei Stufen, hat es sich als vorteilhaft erwiesen, wenn man die zweite Nanofiltration mit einer Membran ausführt, die eine größere Trenngrenze als die Membran der ersten Nanofiltration aufweist.

Konkret empfiehlt es sich die erste Nanofiltration mit einer offenen Membran durchzuführen, vorzugsweise eine mit einer Trenngrenze im Bereich von etwa 500 bis etwa 1.000 Dalton und insbesondere etwa 800 Dalton durchzuführen. Ebenfalls bevorzugt ist es, die Nanofiltration bei einem Druck im Bereich von etwa 10 bis etwa 15 bar zu betreiben. Unter diesen kombinierten Bedingungen wird ein Retentat erhalten, das nur noch etwa 20 % der ursprünglich enthaltenen Milchsäure aufweist. Arbeitet man stattdessen mit geschlossenen Membranen höherer Trenngrenze und/oder höheren oder niedrigeren Drücken werden statt etwa 80 nur noch zwischen etwa 25 und etwa 50 Gew.-% der Milchsäure entfernt, was im Ergebnis dazu führt, dass man die zweite Nanofiltrationsstufe mit höheren Milchsäuregehalten ansteuert und dementsprechend eine im Vergleich niedrigere Trennleistung erreicht.

Vor der zweiten Nanofiltration empfiehlt es sich, dem Retentat 1 die Menge Wasser wieder zuzuführen, die zuvor mit dem Permeat 1 abgeführt wurde. Dadurch wird verhindert, dass der osmotische Druck an dieser Stelle zu hoch wird und die Filtrations-/Reinigungsleistung nicht ausreichend ist. Über die Menge des Diafiltraionswassers kann zudem die Menge an Milchsäure im Retentat 2 gesteuert werden.

Die zweite Nanofiltration wird dann mit einer geschlossenen Membran durchgeführt, die eine Trenngrenze im Bereich von 150 bis 300 Dalton aufweist. Hier empfiehlt es sich nun bei höherem Druck im Bereich von etwa 30 bis etwa 40 bar zu arbeiten. Je nach Milchsäurekonzentration im aufgegebenen Retentat aus der ersten Nanofiltration werden aus der zweiten Stufe Retentate erhalten, die zwischen 0,1 und 0,5 Gew.-% Rest-Milchsäure enthalten und damit Süßmolke praktisch entsprechen oder zumindest nahe kommen.

Der Werkstoff der Nanofiltrationsmembranen kann Edelstahl, Polymerwerkstoffen, Keramik, Aluminiumoxid oder textilen Gewebe darstellen. Es gibt verschiedene Erscheinungsformen der Filterelemente: Kerzenfilter, Flachmembranen, Spiralwickelmembranen, Taschenfilter und Hohlfasermodule, die im Sinne der vorliegenden Erfindung alle grundsätzlich geeignet sind. Vorzugsweise werden jedoch Spiralwickelmembranen aus Polymerwerkstoffen oder Kerzenfilter aus Keramik oder Aluminiumoxid eingesetzt, wobei sich die zweite Ausführungsform für die Nanofiltration besonders bevorzugt erwiesen hat.

Beide Nanofiltrationsschritte können im Sinne der vorliegenden Erfindung "heiß" oder "kalt", d.h. im Temperaturbereich von etwa 10 bis etwa 60 °C durchgeführt werden. Bevorzugt ist es jedoch, bei Temperaturen im niedrigen Bereich von etwa 10 bis etwa 20 °C zu arbeiten.

### UMKEHROSMOSE

Die Umkehrosmose oder Reversosmose ist ein physikalisches Membranverfahren zur Konzentrierung von in Flüssigkeiten gelösten Stoffen, bei der mit Druck der natürliche Osmose-Prozess umgekehrt wird. Im vorliegenden Fall dient dieser Schritt dazu, die Mischung der beiden Permeate P1 und P2 aufzukonzentrieren, damit im folgenden trocknungsschritt weniger Wasser entfernt werden muss.

Das Verfahrensprinzip besteht darin, dass das Medium, in dem die Konzentration eines bestimmten Stoffes verringert werden soll, durch eine halbdurchlässige (semipermeable) Membran von dem Medium getrennt ist, in dem die Konzentration erhöht werden soll. Dieses wird einem Druck ausgesetzt, der höher sein muss als der Druck, der durch das osmotische Verlangen zum Konzentrationsausgleich entsteht. Dadurch können die Moleküle des Lösungsmittels gegen ihre "natürliche" osmotische Ausbreitungsrichtung wandern. Das Verfahren drückt sie in das Kompartiment, in dem gelöste Stoffe weniger konzentriert vorliegen. Typische Drücke für die Umkehrosmose liegen im Bereich von 3 bis 30 bar (Trinkwasserentsalzung) oder bis zu 8o bar (Meerwasserentsalzung).

Die osmotische Membran, die nur die Trägerflüssigkeit (Solvent) durchlässt und die gelösten Stoffe (Solute) zurückhält, muss diesen hohen Drücken standhalten können. Wenn der Druckunterschied das osmotische Gefälle mehr als ausgleicht, passen die Solventmoleküle wie bei einem Filter durch die Membran, während die "Verunreinigungsmoleküle" zurückgehalten werden. Im Gegensatz zu einem klassischen Membranfilter verfügen Osmosemembranen nicht über durchgehende Poren. Vielmehr wandern die Ionen und Moleküle durch die Membran hindurch, indem sie durch das Membranmaterial diffundieren, wie dies durch das Lösungs-Diffusions-Modell beschrieben wird: Der osmotische Druck steigt mit zunehmendem Konzentrationsunterschied. Wird der osmotische Druck gleich dem angelegten Druck, kommt der Prozess zum Stehen. Es liegt dann ein osmotisches Gleichgewicht vor. Ein stetiger Abfluss des Konzentrats kann das verhindern. Beim Konzentratauslass wird der Druck entweder über einen Druckregler kontrolliert oder über einen Druckaustauscher genutzt, um den im Zulauf des Systems benötigten Druck aufzubauen.

### TROCKNUNG

Abschließend können die aus der Umkehrosmose als Retentat erhaltenden Milchsäurekonzentrate entwässert werden. Vorzugsweise kommt dabei die Sprühtrocknung zum Zuge, wobei die Temperatur im Einlass typischerweise etwa 180 bis etwa 260 °C und am Ausgang etwa 80 bis etwa 105 °C beträgt. Die Fraktion bedarf daher keiner Kühlung bevor sie in den Sprühturm gelangt. Temperaturen von 60 bis 70 °C sind dabei sogar bevorzugt, da auf diese Weise die Gefahr verringert wird, dass die Proteine denaturieren. Alternativ können die Produkte auch durch Gefriertrocknung entwässert werden. Die Ausbeute bezogen auf die in der ursprünglichen Sauermolke enthaltende Milchsäure liegt bei 70 bis 90 %, die Reinheit bei 90 bis 95 %.

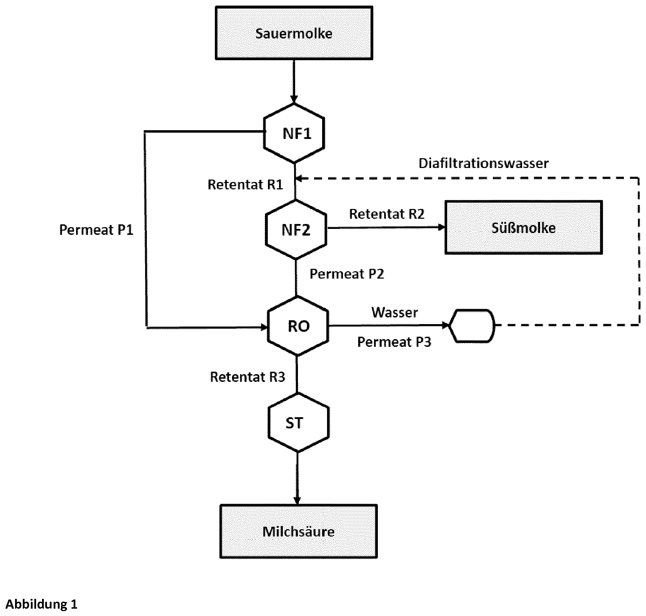

Die Erfindung wird durch die Ansprüche 1-7 definiert und wird im Weiteren durch Ausführungsbeispiele sowie die **Abbildung 1** illustriert, die ein Fließschema des Verfahrens bietet.

### BEISPIELE

### BEISPIEL 1

100 kg Sauermolke mit einem Milchsäureanteil von 0,45 Gew.-% und einem pH-Wert von 4,9 wurden mit einer Flussrate von 5 kg/min einer Nanofiltrationsstufe zugeleitet und dort über eine offene Membran mit einer Trenngrenze von 800 Dalton bei einer Temperatur von 20 °C und einem Druck von 12 bar mit einem Volumenkonzentrierungsfaktor (VKF) von 4 filtriert. Es wurden 75 kg des erstes Permeat P1 erhalten, welches 0,365 Gew.-% Milchsäure aufwies sowie 25 kg des Retentat R1, welches noch 0,7 Gew.-% Milchsäure enthielt. Somit fanden sich 80 % der ursprünglichen Milchsäuremenge im Permeat wieder. Das erste Retentat wurde mit 75 kg Wasser versetzt und einer weiteren Nanofiltrationsstufe zugeführt und dort über eine geschlossene Membran mit einer Trenngrenze von 200 Dalton bei einer Temperatur 20 °C und einem Druck von 35 bar filtriert. Es wurde ein zweites Permeat P2 erhalten, welches 0,18 Gew.-% Milchsäure aufwies, während das zweite Retentat R2 bis auf 0,2 Gew.-% Milchsäure abgereichert worden war und damit die Qualität einer Süßmolke aufwies.

### BEISPIEL 2

100 kg Sauermolke mit einem Milchsäureanteil von 0,45 Gew.-% und einem pH-Wert von 4,9 wurden mit einer Flussrate von 5 kg/min einer Nanofiltrationsstufe zugeleitet und dort über eine offene Membran mit einer Trenngrenze von 800 Dalton bei einer Temperatur von 20 °C, einem Volumenkonzentrierungsfaktor von 4 und einem Druck von 35 bar filtriert. Es wurde ein erstes Permeat P1 erhalten, welches 0,23 Gew.-% Milchsäure aufwies sowie ein erstes Retentat R1, welches 1,1 Gew.-% Milchsäure enthielt. Somit fanden sich in diesem Beispiel nur 50 % der ursprünglichen Milchsäuremenge im Permeat wieder. Das erste Retentat wurde zunächst wieder mit 75 kg Wasser (P3) versetzt und anschließend einer weiteren Nanofiltrationsstufe zugeführt und dort über eine geschlossene Membran mit einer Trenngrenze von 200 Dalton bei einer Temperatur 20 °C und einem Druck von 35 bar filtriert. Es wurde ein zweites Permeat P2 erhalten, welches 0,05 Gew.-% Milchsäure aufwies, während das zweite Retentat R2 bis auf 0,95 Gew.-% Milchsäure im Konzentrat abgereichert worden war, dies entspricht etwa 0,27 Gew.-% Milchsäure der Ursprungslösung und weist damit eine ähnliche Qualität wie Süßmolke auf.

### BEISPIEL 3

100 kg Sauermolke mit einem Milchsäureanteil von 0,45 Gew.-% und einem pH-Wert von 4,9 wurden mit einer Flussrate von 5 kg/min einer Nanofiltrationsstufe zugeleitet und dort über eine geschlossene Membran mit einer Trenngrenze von 200 Dalton bei einer Temperatur von 20 °C, eimem VKF von 4 und einem Druck von 35 bar filtriert. Es wurden 75 kg des ersten Permeates P1 erhalten, welches 0,11 Gew.-% Milchsäure aufwies sowie 25kg des ersten Retentates R1, welches noch 1,47 Gew.-% Milchsäure enthielt. Somit fanden sich in diesem Beispiel nur 25 % der ursprünglichen Milchsäuremenge im Permeat wieder. Das erste Retentat wurde mit 75 kg Wasser (P3) versetzt und einer weiteren Nanofiltrationsstufe zugeführt und dort über eine geschlossene Membran mit einer Trenngrenze von 200 Dalton bei einer Temperatur 20 °C und einem Druck von 35 bar filtriert. Es wurde ein zweites Permeat P2 erhalten, welches 0,09 Gew.-% Milchsäure aufwies, während das zweite Retentat R2 1,2 Gew.-% Milchsäure im Konzentrat aufweist, dies entspricht 0,3 Gew-% in der Ausgangskonzentration. Somit konnte bedingt ähnliche Qualität wie Süßmolke erreicht werden.

### BEISPIEL 4

Die jeweils ersten und zweiten Permeate aus den Beispielen 1 bis 3 wurden einer Umkehrosmoseeinheit zugeführt und dort bis auf einen Restwassergehalt von 25 Gew.-% aufkonzentriert. Anschließend wurden die Konzentrate über einen Wärmeaustauscher auf 90 °C vorgeheizt, auf einen Turm gegeben und bei 180 °C sprühgetrocknet. Die Milchsäure wurde als feines weißes Kristallpulver in einer Reinheit von etwa 85 % erhalten. Etwa 10-20% der Trockenmasse werden durch die Mineralsalze aus der Sauermolke gebildet. Diese Salze sind bedingt durch den niedrigen pH-Wert der Sauermolke auch permeabel an der NF-Membrane

## Patentansprüche

1. Verfahren zur gekoppelten Herstellung von Süßmolke und Milchsäure aus Sauermolke, umfassend die folgenden Schritte:
(a) Bereitstellung einer Sauermolke mit einem Milchsäuregehalt von etwa 0,1 bis etwa 1 Gew.-%;
(b) Nanofiltration der Sauermolke unter Gewinnung eines ersten Permeats P1 und eines ersten Retentats R1;
(c) gegebenenfalls Rückverdünnen des ersten Retentats R1 mit Wasser zum Wiedereinstellen der Ausgangstrockenmasse und Vorbereitung des zweiten Nanofiltrationsschrittes;
(d) Nanofiltration des Retentats R1 unter Gewinnung eines zweiten Permeats P2 und einer Süßmolke als zweitem Retentats R2, wobei eine geschlossene NF-Membran mit einer Trenngrenze im Bereich von 150 bis 300 Dalton einsetzt wird;
(e) Vereinigen der beiden Permeate P1 und P2 und Unterwerfen der Mischung einer Umkehrosmose, unter Erhalt eines im Wesentlichen nur Wasser enthaltende dritten Permeats P3 und eines Milchsäurekonzentrats als drittes Retentat R3.

2. Verfahren nach Anspruch 1, **dadurch gekennzeichnet, dass** man Sauermolke einsetzt, die einen Milchsäuregehalt im Bereich von etwa 0,1 bis 1 Gew.-% aufweist.

3. Verfahren nach mindestens einem der Ansprüche 1 bis 2, **dadurch gekennzeichnet, dass** man die erste Nanofiltration mit einer offenen Membran durchführt.

4. Verfahren nach Anspruch 3, **dadurch gekennzeichnet, dass** man eine offene NF-Membran mit einer Trenngrenze im Bereich von etwa 500 bis etwa 2.000 Dalton einsetzt.

5. Verfahren nach mindestens einem der Ansprüche 1 bis 4, **dadurch gekennzeichnet, dass** man die erste Nanofiltration bei einem Druck im Bereich von etwa 10 bis etwa 15 bar durchführt.

6. Verfahren nach mindestens einem der Ansprüche 1 bis 5, **dadurch gekennzeichnet, dass** man die zweite Nanofiltration bei einem Druck im Bereich von etwa 30 bis etwa 40 bar durchführt.

7. Verfahren nach mindestens einem der Ansprüche 1 bis 6, **dadurch gekennzeichnet, dass** man das Milchsäurekonzentrat anschließend entwässert.

## Claims

1. A process for the coupled production of sweet whey and lactic acid from acid whey, comprising the following steps:
(a) providing acid whey having a lactic acid content of about 0.1 to about 1% by weight;
(b) nanofiltration of the acid whey obtaining a first permeate P1 and a first retentate R1;
(c) optionally, redilution of the first retentate R1 with water to reconstitute the initial dry matter content, and preparation of the second nanofiltration step;
(d) nanofiltration of the retentate R1, obtaining a I second permeate P2 and sweet whey as a second retentate R2, wherein the nanofiltration step is performed using a closed membrane having a cut-off in the range of 150 to 300 Dalton; and
(e) combining the two permeates P1 and P2 and subjecting the mixture to reverse osmosis, obtaining a third permeate P3 which, substantially, only contains water, and a concentrate of lactic acid as a third retentate R3.

2. The process according to claim 1, **characterized in that** acid whey having a lactic acid content in the range of about 0.1 to 1 % by weight is used.

3. The process according to at least one of claims 1 to 2, **characterized in that** the first nanofiltration step is performed using an open membrane.

4. The process according to claim 3, **characterized in that** an open nanofiltration membrane having a cut-off in the range of about 500 to about 2.000 Dalton is used.

5. The process according to at least one of claims 1 to 4, **characterized in that** the the first nanofiltration step is performed at a pressure in the range of about 10 to about 15 bar.

6. The process according to at least one of claims 1 to 5, **characterized in that** the the second nanofiltration step is performed at a pressure in the range of about 30 to about 40 bar.

7. The process according to at least one of claims 1 to 6, **characterized in that** the the lactic acid concentrate is subsequently dehydrated.

## Revendications

1. Procédé de fabrication couplée de lactosérum doux et d'acide lactique à partir de lactosérum acide, comprenant les étapes suivantes :
(a) la préparation d'un lactosérum acide ayant une teneur en acide lactique d'environ 0,1 à environ 1 % en poids ;
(b) la nanofiltration du lactosérum acide avec obtention d'un premier perméat P1 et d'un premier rétentat R1 ;
(c) éventuellement la reconstitution du premier rétentat R1 avec de l'eau pour le réajustement de la masse sèche de départ et la préparation de la deuxième étape de nanofiltration ;
(d) la nanofiltration du rétentat R1 avec obtention d'un deuxième perméat P2 et d'un lactosérum doux en tant que deuxième rétentat R2, une membrane de NF fermée ayant un seuil de séparation dans la plage allant de 150 à 300 Dalton étant utilisée ;
(e) la réunion des deux perméats P1 et P2, et la soumission du mélange à une osmose inverse, avec obtention d'un troisième perméat P3 ne contenant essentiellement que de l'eau, et d'un concentré d'acide lactique en tant que troisième rétentat R3.

2. Procédé selon la revendication 1, **caractérisé en ce qu'**un lactosérum acide qui présente une teneur en acide lactique dans la plage allant d'environ 0,1 à 1 % en poids est utilisé.

3. Procédé selon au moins l'une quelconque des revendications 1 à 2, **caractérisé en ce que** la première nanofiltration est réalisée avec une membrane ouverte.

4. Procédé selon la revendication 3, **caractérisé en ce qu'**une membrane de NF ouverte ayant un seuil de séparation dans la plage allant d'environ 500 à environ 2 000 Dalton est utilisée.

5. Procédé selon au moins l'une quelconque des revendications 1 à 4, **caractérisé en ce que** la première nanofiltration est réalisée à une pression dans la plage allant d'environ 10 à environ 15 bar.

6. Procédé selon au moins l'une quelconque des revendications 1 à 5, **caractérisé en ce que** la deuxième nanofiltration est réalisée à une pression dans la plage allant d'environ 30 à environ 40 bar.

7. Procédé selon au moins l'une quelconque des revendications 1 à 6, **caractérisé en ce que** le concentré d'acide lactique est ensuite déshydraté.
